# EUROPEAN PATENT APPLICATION

(11) **EP 1 728 488 A1**
(43) Date of publication of application: **06.12.2006**
(21) Application number: 06011243.0
(22) Date of filing: 31.05.2006
(51) Int. Cl.: A61F 2/16

(54) **Intraocular lens injector**

(30) Priority: 31.05.2005 JP 2005159180
(71) Applicant: Nidek Co., Ltd., Gamagori-shi, Aichi-ken 443-0035 (JP)
(72) Inventor: Sunada, Tsutomu, Gamagori-shi Aichi-ken 443-0035 (JP); Nagasaka, Shinji, Gamagori-shi Aichi-ken 443-0035 (JP); Oda, Haruo, Gamagori-shi Aichi-ken 443-0035 (JP)
(74) Representative: Materne, Jürgen

(57) **Abstract**

An intraocular lens injector (1) for injecting a foldable intraocular lens (40) in an aye, comprises: a substantially cylindrical main unit (10, 20) including a substantially cylindrical insertion part (11) which is insertable through an incision made in the eye, the main unit being adapted to internally hold the intraocular lens and release the intraocular lens to the outside of the injector through the insertion part; a push unit (30) inserted in the main unit to push the intraocular lens to the outside through the insertion part, the push unit being disposed to be unrotatable relative to the main unit but movable in an axial direction of the main unit; and a rotatable member (50) disposed to be rotatable relative to the main unit.

## Description

### BACKGROUND OF THE INVENTION

### 1. Field of the Invention

The present invention relates to an intraocular lens injector for injecting an intraocular lens into an eye of a patient.

### 2. Description of Related Art

As one of operative treatments for cataract, generally used is a method of removing a crystalline lens from an eye of a patient and then injecting an intraocular lens in place of the crystalline lens. To inject the intraocular lens, the following steps are performed: first making an incision in the eye; fragmenting and aspirating a clouded crystalline lens through the incision by for example an ultrasonic cataract-surgery device (a phaco-emulsification device); and then injecting the intraocular lens into the eye through the incision to implant it in place of the crystalline lens.

If a large incision is made, it may become a burden on the eye and also cause astigmatism of the eye after the operation. To prevent such disadvantages, an intraocular lens injector is used to inject a foldable intraocular lens into an eye through a smaller incision. In this injector, the foldable intraocular lens held in a housing of the injector is pushed toward the tip of the injector while being folded into a smaller shape. Thereafter, the folded intraocular lens is pushed out of the tip of the injector inserted in the eye through the incision and is spread (unfolded) and placed in the eye.

When the intraocular lens is to be injected and placed in the eye with the above injector, it is necessary to adjust the direction of spreading (unfolding) the intraocular lens within the eye in order to prevent the intraocular lens from coming into contact with a crystalline lens capsule and others.

### BRIEF SUMMARY OF THE INVENTION

The present invention has been made in view of the above circumstances and has an object to provide an intraocular lens injector capable of adjusting the direction of spreading (unfolding) a folded intraocular lens within an eye while the intraocular lens is being injected to be placed in the eye.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the purpose of the invention, there is provided an intraocular lens injector for injecting a foldable intraocular lens in an aye, comprising: a substantially cylindrical main unit including a substantially cylindrical insertion part which is insertable through an incision made in the eye, the main unit being adapted to internally hold the intraocular lens and release the intraocular lens to the outside of the injector through the insertion part; a push unit inserted in the main unit to push the intraocular lens to the outside through the insertion part, the push unit being disposed to be unrotatable relative to the main unit but movable in an axial direction of the main unit; and a rotatable member disposed to be rotatable relative to the main unit.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1A is a schematic plan view of an intraocular lens injector in a preferred embodiment of the present invention;
Fig. 1B is a schematic side view of the intraocular lens injector;
Fig. 2 is a schematic structural view of an intraocular lens;
Fig. 3A is a perspective view of a lens holding unit viewed from behind;
Fig. 3B is a plan view of the lens holding unit;
Fig. 4 is a schematic structural view of a main unit and a push unit;
Fig. 5 is a schematic sectional view of the main unit and the push unit; and
Fig. 6 is a schematic sectional view of a modification of the main unit.

### DETAILED DESCRIPTION OF THE PREFERRED EMBODIMENTS

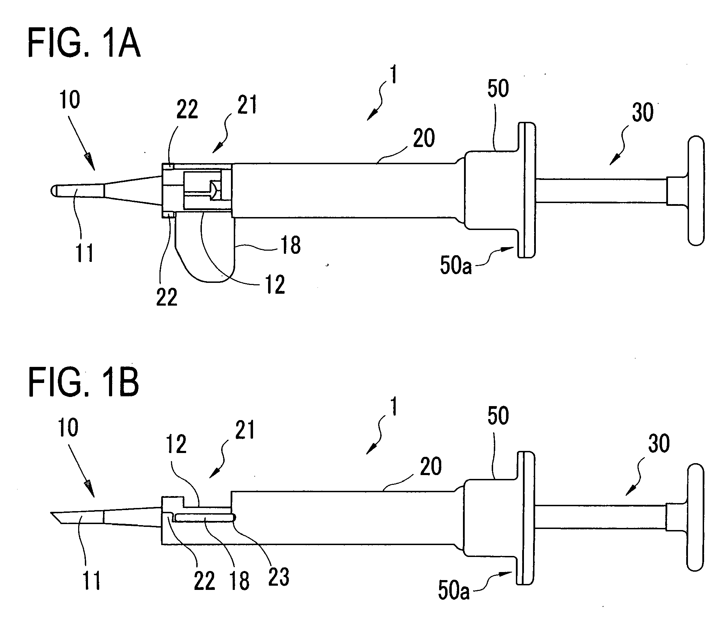

A detailed description of a preferred embodiment of the present invention will now be given referring to the accompanying drawings. Figs. 1A and 1B are schematic structural views of an intraocular lens injector 1 in the present embodiment; Fig. 1A is a plan view and Fig. 1B is a side view.

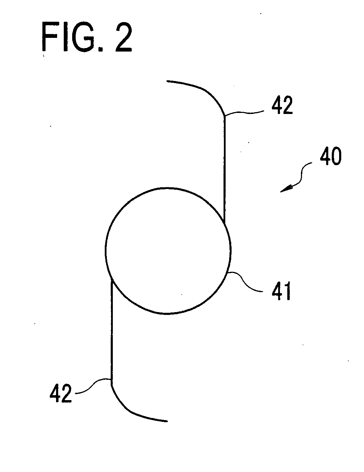

The injector 1 includes, in order of insertion into an eye, a substantially cylindrical lens holding unit (a lens cartridge) 10 provided with a substantially cylindrical (tubular) insertion part 11 which is insertable in an incision made in the eye and a setting part 12 in which an intraocular lens (hereinafter, IOL) 40 (see Fig. 2) is to be placed (set), a substantially cylindrical (tubular) main unit 20 in which the lens holding unit 10 is mounted, and a push unit 30 inserted in the main unit 20 and the lens holding unit 10. This push unit 30 is used to push out the IOL 40 placed in the setting part 12 of the lens holding unit 10 through a front end (a tip end) of the insertion part 11 to the outside. A rotatable member 50 is fitted on a rearward part of the main unit 20 (opposite to the front end) so that the rotatable member 50 is rotatable with respect to the main unit 20 (the details thereof will be mentioned later).

Fig. 2 is a schematic structural view of the IOL 40. This IOL 40 includes an optical portion 41 having a predetermined refractive power and a pair of support portions 42 which support the optical portion 41 in an eye. The IOL 40 in the present embodiment is made of a material commonly used for an optical portion of a foldable soft intraocular lens, for example, a monomeric material such as hydroxyethyl methacrylate (HEMA) or a composite material such as acrylic ester and methacrylate ester. The support parts 42 are made of a material commonly used for a support portion of an intraocular lens such as polymethyl methacrylate (PMMA).

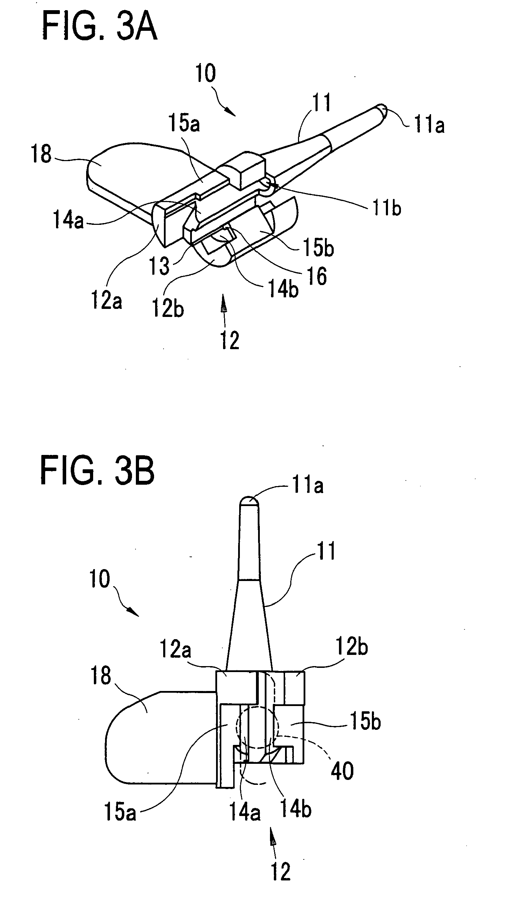

Figs. 3A and 3B are schematic structural views of the lens holding unit 10. Specifically, Fig. 3A is a perspective view of the unit 10 viewed from behind (opposite to the tip end), Fig. 3B is a plan view. The lens holding unit 10 is entirely made of resin and is a disposable type which is to be thrown away after one use.

The insertion part 11 of the lens holding unit 10 is formed with a diametrical taper so that its diameter becomes smaller (narrower) toward a tip end 11a to be inserted in an incision made in an eye. With this structure, the folded IOL 40 is further folded into a smaller shape while passing through the hollow of the insertion part 11, and then the IOL 40 is pushed out (released) through the tip end 11a to the outside.

In the lens holding unit 10, the setting part 12 is arranged on a side opposite to the tip end 11a of the insertion part 11 and adapted to be opened and closed about a hinge 13. The setting part 12 includes a fixed part 12a fixed to the insertion part 11 and a movable part (an openable and closable part) 12b connected with the fixed part 12a with the hinge 13. The fixed part 12a has a setting surface 14a and the movable part 12b has a setting surface 14b. The IOL 40 is placed (set) on the setting surfaces 14a and 14b while the setting part 12 (i.e., the fixed part 12a and the movable part 12b) is opened. In this state, the IOL 40 is unfolded.

Thereafter, when the setting part 12 (i.e., the fixed part 12a and the movable part 12b) is closed, the shape (in section) of the setting surfaces 14a and 14b becomes substantially equal to the shape of a semicircular opening 11b of the insertion part 11 opening into the setting part 12. Thus, the IOL 40 placed on the setting surfaces 14a and 14b is correspondingly folded along the shape of the setting surfaces 14a and 14b. To be more specific, the setting surfaces 14a and 14b have a curve that allows the IOL 40 to be held in an unfolded state while the setting part 12 is opened and in a folded state when the setting part 12 is closed.

The fixed part 12a and the movable part 12b are provided with covers 15a and 15b respectively. These covers 15a and 15b are formed to cover the setting surfaces 14a and 14b above them when the setting part 12 (the fixed part 12a and the movable part 12b) is closed. The cover 15b is provided at an end facing the cover 15a with a protrusion 16 extending downward toward the setting surfaces 14a and 14b. This protrusion 16 serves to restrict improper folding of the IOL 40 in a direction other than an opening and closing direction of the setting part 12 corresponding to a folding direction of the IOL 40.

A lug 18 formed of a flat plate is provided on the side of the fixed part 12a. This lug 18 is used as a grip portion to be held by a user with his hand (fingers) to mount the lens holding unit 10 in the main unit 20.

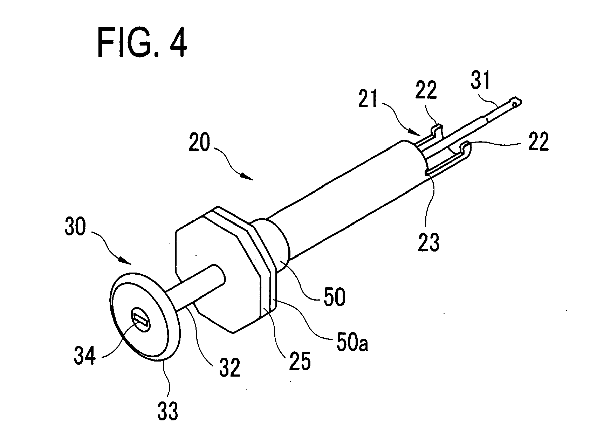

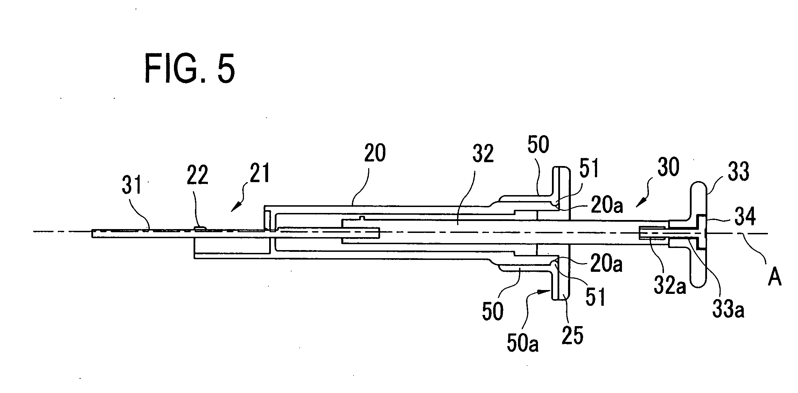

Figs. 4 and 5 are schematic structural views of the main unit 20 and the push unit 30; Fig. 4 is a perspective view of them viewed from behind and Fig. 5 is a sectional side view of them. The main unit 20 is provided in the forward part with a mounting part 21 in which the lens holding unit 10 is to be mounted. This mounting part 21 is of a substantially half shape (in diametrical section) of the main unit 20 and is formed, in the front, with protrusions 22 in symmetrical relation with respect to a central axis A of the main unit 20. The protrusions 22 are positioned slightly above the central axis A and have a distance therebetween slightly narrower (shorter) than the inner diameter of the main unit 20. With this structure, the protrusions 22 serve to guide the setting part 12 in a closing motion when the lens holding unit 10 is mounted in the mounting part 21. Further, the protrusions 22 engage the lens holding unit 10 to prevent the unit 10 from easily becoming detached from the mounting part 21. As with the protrusions 22, right and left edges of the mounting part 21 also serve to guide the setting part 12 in the closing motion.

The mounting part 21 is formed with a recess 23 in the left back (a right end in Fig. 1B). This recess 23 serves to guide the setting part 12 in the closing motion when the lens holding unit 10 is mounted in the mounting part 21. Further, the recess 23 receives part of a rear edge of the lug 18 in engagement relation to prevent the lens holding unit 10 from easily becoming detached. In the present embodiment, the mounting part 21 is also formed with a recess 23 in the right back. Accordingly, another lens holding unit 10 provided with the fixed part 12a and the movable part 12b arranged in counterchanged positions (the lug 18 is correspondingly formed on an opposite side) can be used.

The substantially cylindrical rotatable member 50 having a flared finger rest 50a is attached to the rearward part of the main unit 20 which is inserted in the rotatable member 50. This rotatable member 50 is rotatable about the central axis A with respect to the main unit 20. The main unit 20 is further provided with a recess 20a formed along the outer periphery of the rearward part of the main unit 20. Correspondingly, the rotatable member 50 is provided, along the inner periphery thereof, with a projection 51 engageable with the recess 20a of the main unit 20. Since the projection 51 of the rotatable member 50 is engaged in the recess 20a of the main unit 20, the rotatable member 50 is prevented (inhibited) from moving in a direction of the central axis A with respect to the main unit 20. In other words, the recess 20a (and the projection 51) functions as a stopper to hold the rotatable member 50 so as to be rotatable with respect to the main unit 20 but immovable in the direction of the central axis A.

The push unit 30 is inserted in the main unit 20 so as to be unrotatable relative to the main unit 20 but movable in the direction of the central axis A. This push unit 30 includes a push portion (rod) 31, a shaft portion 32, and a push member (thumb rest) 33. The push portion 31 is fixedly joined with a front end of the shaft portion 32 which is unrotatable relative to the main unit 20 but movable in the direction of the central axis A. The push member 33 is rotatably attached to a rear end of the shaft portion 32. Specifically, the rear end of the shaft portion 32 is formed with a screw hole 32a in which a screw 34 is tightened through an opening 33a centrally formed in the push member 33. This opening 33a has an inner diameter slightly larger than an outer diameter of the screw 34, thus allowing the push member 33 to rotate about the screw 34. A stopper 25 is fitted in a rear opening of the main unit 20 so that the push unit 30 (the shaft portion 32) is inserted in the stopper 25, thus preventing the push unit 30 from coming off the main unit 20.

An operation of the injector 1 constructed as above will be explained below.

A user puts (sets) the IOL 40 in the setting part 12 (i.e., on the setting surfaces 14a and 14b) of the lens holding unit 10 and then holds the lens holding unit 10 by grasping the lug 18 with one hand (normally, his/her thumb and index finger). Thereafter, the user engages the rear end of the lug 18 of the lens holding unit 10 in the recess 23 of the mounting part 21, and pushes down the setting part 12 into the mounting part 21 so as to press the bottom of the mounting part 12 against the protrusions 22. Accordingly, the lens holding unit 10 with the fixed part 12a and the movable part 12b being closed is properly mounted in the mounting part 21.

In the closed state of the setting part 12 (the fixed part 12a and the movable part 12b), the width (distance) of the setting surfaces 14a and 14b in diametrical section is narrower. At this time, the IOL 40 placed on the setting surfaces 14a and 14b is subjected to stress and correspondingly folded along the curved setting surfaces 14a and 14b.

After mounting the lens holding unit 10 in the main unit 20 (the mounting part 21), the user performs the following operation to inject the IOL 40 into the eye by manipulation of the injector 1. To hold the injector 1, for example, the user may hold the thumb of one hand against the push member 33 while holding the rotatable member 50 between other fingers (normally, the index finger and middle finger) in a manner similar to hold a syringe. At this time, the user should put the fingers on the finger rest 50a to easily hold the rotatable member 50. And the user puts the other hand on the main unit 20 for stabilization.

When the push member 33 is pushed by the user, the shaft portion 32 and the push portion 31 are moved forward, and the push portion 31 pushes the IOL 40 placed on the setting part 12 into the insertion part 11. As pushed into the insertion part 11, the IOL 40 is folded (rolled) along the inner surface of the insertion part 11. When the push member 33 is further pushed, moving the shaft portion 32 and the push portion 31 forward, the IOL 40 folded up into a smaller shape will be pushed (released) out of the insertion part 11 through the tip end 11a.

The IOL 40 will then be spread (unfolded) gradually from a portion pushed (released) out of the insertion part 11 through the tip end 11a. At this time, the user who holds the rotatable member 50 with one hand may rotate the main unit 20 with the other hand at a desired timing. In association with the rotation of the main unit 20, the shaft portion 32 and the push portion 31 are together rotated with respect to the push member 33 on which the thumb is pressed. Accordingly, the direction of spreading (unfolding) the IOL 40 is also rotated. This makes it possible for the user to adjust the direction of spreading (unfolding) the IOL 40 within the eye while pushing out the IOL 40.

In the present embodiment, the rotatable member 50 is made rotatable with respect to the main unit 20 and the push member 33 is rotatable with respect to the shaft portion 32 (the push portion 31). Alternatively, only the rotatable member 50 may be made rotatable. Although the rotatable member 50 is rotatably fitted on the outer periphery of the main unit 20, the rotatable member 50 only has to be provided at any portion of the injector 1 to be held with user's fingers and also arranged to be rotatable with respect to the push member 31 (the shaft portion 32). For instance, the main unit 20 may be made of separate parts one of which is rotatable to be used as the rotatable member 50.

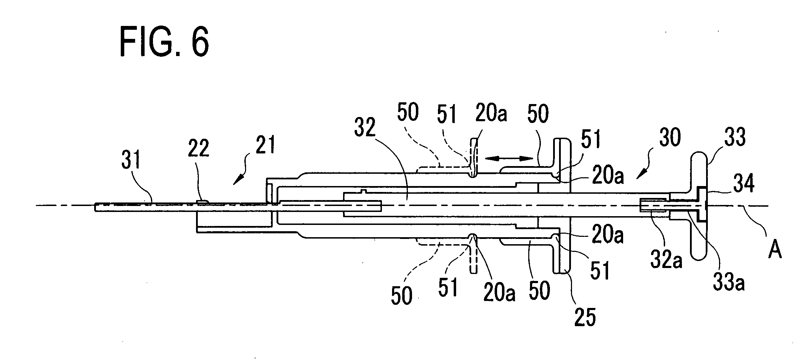

In the present embodiment, furthermore, the rotatable member 50 is rotatably held on the rearward part of the main unit 20. Alternatively, the rotatable member 50 may be held at one of plural different positions of the main unit 20 along the central axis A. For instance, the rotatable member 50 may be held at either of two positions as shown in Fig. 6. In this case, the main unit 20 is formed with two recesses 20a spaced apart along the central axis A, which are engageable with the projection 51 of the rotatable member 50. This structure makes it possible to selectively hold the rotatable member 50 at a desired position so that the user places the rotatable member 50 at a position making it easy to manipulate. In Fig. 6, two positions for holding the rotatable member 50 are provided, that is, two recesses 20a are formed. However, more than two positions (recesses) may also be provided.

Furthermore, although the lens holding unit 10 provided with the insertion part 11 and the setting part 12 is removably mounted in the main unit 20, the main unit (the main body) may be configured to integrally have the insertion part and the setting part.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injector (1) for injecting a foldable intraocular lens (40) in an aye, comprising:
a substantially cylindrical main unit (10, 20) including a substantially cylindrical insertion part (11) which is insertable through an incision made in the eye, the main unit being adapted to internally hold the intraocular lens and release the intraocular lens to the outside of the injector through the insertion part;
a push unit (30) inserted in the main unit to push the intraocular lens to the outside through the insertion part, the push unit being disposed to be unrotatable relative to the main unit but movable in an axial direction of the main unit; and
a rotatable member (50) disposed to be rotatable relative to the main unit.

2. The injector according to claim 1, wherein the push unit includes a shaft portion (32) disposed to be unrotatable relative to the main unit but movable in the axial direction and a push member (33) rotatably provided at an end of the shaft portion.

3. The injector according to claim 1 or 2, wherein the main unit includes a stopper (20a) for holding the rotatable member to be rotatable relative to the main unit but immovable in the axial direction.

4. The injector according to claim 3, wherein the stopper is provided in the main unit at each of a plurality of different positions in the axial direction.

5. The injector according to one of claims 1 to 4, wherein the rotatable member is a substantially cylindrical member provided with a flared finger rest (50a).
